# EUROPÄISCHE PATENTANMELDUNG

(11) **EP 2 918 201 A1**
(43) Veröffentlichungstag der Anmeldung: **16.09.2015**
(21) Anmeldenummer: 14405023.4
(22) Anmeldetag: 13.03.2014
(51) Int. Cl.: A47J 31/60

(54) **Verfahren zum Reinigen und/oder Desinfizieren eines milchführenden Systems einer Kaffeemaschine sowie Kaffeemaschine**

(71) Anmelder: JURA ELEKTROAPPARATE AG, 4626 Niederbuchsiten (CH)
(72) Erfinder: Büttiker, Philipp, 4625 Oberbuchsiten (CH); Rütti, Pascal, 4623 Neuendorf (CH)
(74) Vertreter: EGLI-EUROPEAN PATENT ATTORNEYS

(57) **Zusammenfassung**

Es wird ein Verfahren zum Reinigen und/oder Desinfizieren eines milchführenden Systems (90) einer Kaffeemaschine zum Herstellen von Kaffeemischgetränken und eine Kaffeemaschine zum Durchführen des Verfahrens angegeben. Mit dem Ziel, eine gut automatisierbare und vollständige Reinigung des gesamten milchführenden Systems (90) zu erreichen, ist erfindungsgemäss vorgesehen, dass die Milchzufuhrleitung zum Durchführen des Reinigungsvorganges vom Milchbehälter (7) getrennt und mit einem ersten Behälter (10) verbunden wird und ein zweiter Behälter (11) unterhalb eines Milchauslasses (12) einer Milchzubereitungseinrichtung (13) des milchführenden Systems (90) angeordnet wird. Es wird dann Fluid zu einem Fluideinlass (41) zugeführt, um die reinigungsaktive und/oder desinfizierende Substanz durch die Milchzufuhrleitung (80) und einen Milchauslass (12) in den zweiten Behälter (11) zu fördern. Anschliessend ist vorgesehen, Wasser zu dem Lufteinlass (51) zu leiten und den Fluideinlass (41) zu verschliessen, um so das milchführende System (90) zu spülen.

## Beschreibung

Die vorliegende Erfindung betrifft ein Verfahren zum Reinigen und/oder zum Desinfizieren eines milchführenden Systems einer Kaffeemaschine sowie eine Kaffeemaschine zum Durchführen des Verfahrens.

Aus dem Stand der Technik sind Kaffeemaschinen, insbesondere sogenannte Kaffeevollautomaten bekannt, welche zum Herstellen sogenannter Kaffeemischgetränke geeignet sind. Derartige Kaffeemischgetränke wie z. B. Cappuccino, Latte Macchiato und dergleichen weisen hierbei einen mehr oder weniger hohen Milchanteil auf.

Um den Bedienkomfort zu erhöhen, ist es seit einiger Zeit üblich, derartige Kaffeemaschinen mit Milchzubereitungseinrichtungen zu versehen, welche zugeführte Milch erhitzen und/oder zum Erzielen eines Aufschäumeffektes mit Dampf und/oder Luft vermischen. Die Milch wird hierzu in einem separaten, teilweise gekühlten Behälter zur Verfügung gestellt und über eine Milchzufuhrleitung, beispielsweise einen Milchschlauch an einen Milcheinlass der Milchzubereitungseinrichtung zugeführt.

Die Milchzubereitungseinrichtung weist des Weiteren einen Fluideinlass sowie einen Lufteinlass auf. Der Lufteinlass wird belüftet, d. h. mit der Umgebungsatmosphäre verbunden, während der Fluideinlass zum Fördern und/oder Erwärmen der Milch mit einem Fluid versorgt wird, beispielsweise mit Dampf. Durch den Venturi-Effekt wird hierbei die Milch aus dem Milchvorratsbehälter angezogen, gegebenenfalls mit dem Dampf und/oder mit Luft vermischt und an einem Milchauslass der Milchzubereitungseinrichtung ausgegeben.

Da Milch für gewöhnlich eine hohe Keimzahl aufweist und relativ schnell verdirbt, ist es bei den herkömmlichen Systemen vorgesehen, nach jedem Milchbezug eine Spülung und mindestens einmal täglich eine Reinigung und/oder Desinfizierung, beispielsweise mit einem keimabtötendem Reinigungsmittel durchzuführen. Derartige Reinigungsabläufe sind für eine Bedienperson relativ kompliziert. Der Anwender muss mehrmals während eines derartigen Reinigungs- und/oder Desinfizierungsablaufes Handhabungen an der Kaffeemaschine durchführen.

Des Weiteren sind weitgehend automatisierte Reinigungsabläufe für milchführende Systeme in Kaffeemaschinen bekannt. Derartige automatisierte Reinigungs- und/oder Desinfizierungsvorgänge gemäss dem Stand der Technik weisen den Nachteil auf, dass das milchführende System nicht vollständig gereinigt werden kann. Zudem ist oftmals ein Kreislauf für das Reinigungsmittel vorgesehen, d. h. dasselbe, nach einem ersten Reinigungsvorgang bereits mit Milchresten verunreinigte Reinigungsmittel wird bei folgenden Reinigungsvorgängen wiederholt durch den Milch-Fluidpfad geleitet und wird deshalb im Laufe der Zeit immer stärker kontaminiert (mit wachsender Anzahl aufeinanderfolgender Reinigungsvorgänge).

Aus der DE 20 2010 010 509 U1 ist eine Reinigungseinheit für eine Kaffeemaschine bekannt, welche ein milchführendes System aufweist. Hierbei ist eine fest installierte Dosier- und Reinigungseinheit vorgesehen, was das Bauvolumen einer derartigen Kaffeemaschine vergrössert und den Platzbedarf erhöht. Da ein Umschalten von einem Milchpfad auf einen Reinigungsfluidpfad vorgesehen ist, ist hierbei ab dem Umschaltventil in Richtung der Milchzufuhr, d. h. in Richtung des Milchbehälters keine vollständige Reinigung und Durchspülung des milchführenden Systems möglich.

Aus der EP 1 872 698 B1 ist ein Spülvorgang für das milchführende System eines Automaten für Heissgetränke bekannt. Hierbei wird Wasser zum Spülen durch den Luftzufuhrschlauch der Milchaufschäumeinrichtung geleitet. Es erfolgt hierbei nur ein Durchspülen mit heissem Wasser, d. h. es wird kein Reiniger zugesetzt. Der eigentliche Zufuhrschlauch für die Milch wird hierbei nicht gespült, sondern es ist vielmehr eine Entsorgung des Milchzufuhrschlauches in bestimmten Zeitintervallen vorgesehen.

Aus der EP 1 656 863 B1 ist eine besondere Ausgestaltung des Milch-Dampf-Mischers einer Milchzubereitungseinrichtung bekannt. Zum Spülen des Mischers ist ein Zuleiten eines Reinigungsfluids vorgesehen. Ein vollständiges Spülen des milchführenden Systems, d. h. inklusive der Zuleitungsschläuche, ist hierbei nicht möglich.

Aus der EP 2 363 051 A1 ist ein Kaffeevollautomat mit einem sogenannten Milchmodul bekannt, mit welchem aufgeschäumte Milch erzeugt werden kann. Der Kaffeevollautomat weist zum Reinigen wiederum ein eigenes Reinigungsmittelreservoir auf, was den Platzbedarf erhöht. Im Reinigungspfad ist ein Rückschlagventil vorgesehen, welches ein Trennen des Milchvorratsbehälters inklusive eines Stücks seiner Zuleitung vorsieht. Ein vollständiges Spülen des gesamten milchführenden Systems ist hierbei nicht möglich.

Aus der EP 2 277 419 B1 ist ein Getränkeautomat bekannt, bei welchem eine Milchzuführungsleitung in einen Milchbehälter zur gemeinsamen Reinigung integriert ist. Der Milchbehälter inklusive der Milchzuführungsleitung muss hierbei extern gereinigt werden, d. h. es ist kein Desinfizieren bzw. Reinigen des gesamten milchführenden Systems vor Ort möglich, was den Bedienungsaufwand erhöht.

Es ist die Aufgabe der vorliegenden Erfindung, ein Verfahren zum Reinigen und/oder Desinfizieren eines milchführenden Systems einer Kaffeemaschine zum Herstellen von Kaffeemischgetränken anzugeben, welches es auf einfache Weise ermöglicht, das gesamte milchführende System auf einfache Weise zu Reinigen und/oder zu Desinfizieren. Es ist eine weitere Aufgabe der vorliegenden Erfindung, eine Kaffeemaschine zum Herstellen von Kaffeemischgetränken anzugeben, welche ein milchführendes System aufweist, das auf einfache Art vollständig gereinigt und/oder desinfiziert werden kann.

Die Aufgabe wird gelöst durch ein Verfahren mit den Merkmalen des unabhängigen Anspruches 1 sowie durch eine Kaffeemaschine mit den Merkmalen des Anspruchs 8.

Gemäss dem erfindungsgemässen Verfahren zum Reinigen und/oder Desinfizieren eines milchführenden Systems (90) einer Kaffeemaschine zum Herstellen von Kaffeemischgetränken weist das milchführende System Folgendes auf:
- eine Milchzubereitungseinrichtung mit einem Milchauslass sowie einem Fluideinlass, einem Lufteinlass und einem Milcheinlass, die zum Fördern von Milch vom Milcheinlass zum Milchauslass unter Verwendung des Venturi-Effekts ausgebildet ist; und
- eine Milchzufuhrleitung, die an einem Ende mit dem Milcheinlass verbunden ist und an einem anderen Ende wahlweise mit einem Milchbehälter verbindbar ist.

Das erfindungsgemässe Verfahren weist hierbei die folgenden vorbereitenden Verfahrensschritte auf:
- Trennen der Milchzufuhrleitung vom Milchbehälter und Verbinden der Milchzufuhrleitung mit einem ersten Behälter, der eine reinigungsaktive und/oder desinfizierende Substanz enthält; und
- Positionieren eines zweiten Behälters unterhalb des Milchauslasses.

Das Verfahren weist dann die folgenden Verfahrensschritte in dieser Reihenfolge auf:
- Zuführen von Fluid, vorzugsweise von Dampf, zu dem Fluideinlass, um die reinigungsaktive und/oder desinfizierende Substanz durch die Milchzufuhrleitung und den Milchauslass in den zweiten Behälter zu fördern, um so das milchführende System zu reinigen und/oder zu desinfizieren; und
- Zuführen von Wasser zu dem Lufteinlass und verschliessen des Fluideinlasses, um Wasser durch die Milchzufuhrleitung in den ersten Behälter und um Wasser durch den Milchauslass in den zweiten Behälter zu fördern, um so das milchführende System zu spülen.

Der besondere Vorteil des erfindungsgemässen Verfahrens liegt darin, dass bei nur minimaler Benutzerinteraktion das gesamte milchführende System, d. h. die gesamte Milchzubereitungseinrichtung inklusive der Milchzufuhrleitungen, insbesondere eines Milchschlauches oder dergleichen, vollständig und auf einfache Weise mit einem entsprechenden Fluid gespült werden kann, welches reinigend und/oder desinfizierend wirkt. Durch die Unterteilung in ersten und zweiten Behälter, welche nicht in einer Austauschverbindung stehen, wird ausserdem das Reinigungs- bzw. Desinfektionsfluid nicht im Kreislauf durch die Milchzubereitungseinrichtung und das weitere milchführende System geleitet. Vielmehr wird verunreinigtes (kontaminiertes) Reinigungs- bzw. Desinfektionsfluid im zweiten Behälter aufgefangen und somit das saubere Reinigungs- bzw. Desinfektionsfluid von dem verunreinigten Reinigungs- bzw. Desinfektionsfluid klar voneinander getrennt. Hierdurch wird die Reinigungs- und Desinfektionswirkung merklich verbessert und gleichzeitig das gesamte System, welches die Milch führt, effektiv gereinigt.

Das Ansaugen des Reinigungs- bzw. Desinfektionsfluid, d. h. der fertig gemischten reinigungsaktiven und/oder desinfizierenden Substanz, erfolgt hierbei in dem ersten Verfahrensschritt, in welchem Fluid und vorzugsweise Dampf zu dem Fluideinlass geführt wird, mittels des Venturi-Prinzips. Die reinigungsaktive und/oder desinfizierende Substanz wird also von dem Fluidstrom, vorzugsweise von dem Dampfstrom durch die an den ersten Behälter angeschlossene Milchzufuhrleitung angesaugt und durch den Milchauslass gefördert, um so das System zu reinigen. Indem im zweiten Verfahrensschritt dann Wasser zu dem Lufteinlass geführt wird und der Fluideinlass (gleichzeitig oder vorher) verschlossen wird, wird dann Spülwasser (in der Regel Trinkwasser) in die Milchzubereitungseinrichtung geleitet und verzweigt sich dort. Dadurch werden sowohl der Milchauslass als auch die Milchzufuhrleitung gespült und von der reinigungsaktiven und/oder desinfizierenden Substanz befreit.

Im Anschluss an die Durchführung des erfindungsgemässen Verfahrens ist somit das milchführende System dahingehend wieder einsatzbereit, dass durch ein einfaches Umstecken der Milchzufuhrleitung vom ersten Behälter zum Milchbehälter eine entsprechende Milchzubereitung wieder stattfinden kann.

In einer Ausführungsform des erfindungsgemässen Verfahrens ist vorgesehen, dass das Verfahren nach dem Verfahrensschritt des Zuführens von Wasser zu dem Lufteinlass und des Verschliessens des Fluideinlasses, d. h. nach dem Verfahrensschritt des Spülens, zusätzlich den Verfahrensschritt aufweist, wonach Dampf zu dem Lufteinlass zugeführt wird und insbesondere Dampf zu dem Lufteinlass stossweise zugeführt wird, und wonach der Fluideinlass verschlossen wird, um das Wasser aus der Milchzufuhrleitung und dem Milchauslass auszutreiben.

Durch einen derartigen Dampfstoss nach Beendigung des Spülvorganges kann restliches Spülwasser sicher aus dem gesamten milchführenden System ausgetrieben werden, und ein nachfolgender Bezug von Milch mit Zubereitung in dem milchführenden System kann ohne eine möglicherweise unerwünschte Verdünnung der Milch mit Wasser stattfinden.

Gemäss einem weiteren Aspekt der Erfindung ist vorgesehen, dass das Verfahren vor dem ersten Verfahrensschritt, d. h. vor dem Verfahrensschritt des Zuführens von Fluid zu dem Fluideinlass zusätzlich den Verfahrensschritt aufweist, wonach Wasser, vorzugsweise warmes Wasser (z.B. mit einer Temperatur von 20-40 °C) in den ersten Behälter zugeführt wird und vorzugsweise automatisch in den ersten Behälter zugeführt wird, um die reinigungsaktive und/oder desinfizierende Substanz zu aktivieren.

Dies ist insbesondere von Vorteil, wenn die Reinigung bzw. Desinfektion mittels eines Reinigungskonzentrats oder eines Reinigers in Tablettenform stattfinden soll, welcher zunächst durch entsprechende Verdünnung einsatzbereit gemacht ("aktiviert") werden muss. Indem eine derartige Verdünnung mittels einer Wasserzufuhr automatisiert stattfindet, kann der Vorgang merklich vereinfacht und der Nutzerkomfort erhöht werden.

Gemäss einem weiteren Aspekt der Erfindung ist vorgesehen, dass der erste Behälter und der zweite Behälter ineinander geschachtelt und vorzugsweise integral ausgebildet sind.

Mit anderen Worten: Der zweite Behälter ist vorzugsweise innerhalb des ersten Behälters angeordnet, steht mit diesem jedoch nicht in direkter Flüssigkeitskommunikation. Beispielsweise kann es vorgesehen sein, dass der Behälter als integraler Behälter mit mehreren Kammern ausgebildet ist, wobei eine Behälterkammer den ersten Behälter bildet und eine weitere Behälterkammer den zweiten Behälter bildet. Hierbei sind in den Behältern dann Trennwände eingelassen, welche eine Trennung der einzelnen Behälterkammern voneinander ermöglichen. Durch das Vorsehen eines eigenen derartigen Reinigungsbehälters, welcher besonders einfach zu handhaben ist, kann der Aufwand einer Bedienperson weiter verringert werden und das erfindungsgemässe Verfahren einfach und sicher durchgeführt werden.

Es ist hierbei möglich, den Behälter mit einer an die entsprechende Kaffeemaschine angepassten Form und gegebenenfalls zusätzlich mit einer Positionierhilfe auszustatten, sodass verhindert wird, dass der zweite Behälter (bzw. die zweite Behälterkammer) fehlpositioniert wird und sich nicht unterhalb des Milchauslasses befindet.

Es versteht sich hierbei von selbst, dass der zweite Behälter nach oben hin offen ausgebildet ist, sodass bei einer entsprechenden Positionierung des zweiten Behälters unterhalb des Milchauslasses die reinigungsaktive Substanz und/oder das Spülwasser in diesem zweiten Behälter ungehindert einfliessen kann. Der Einfachheit halber wird auch der erste Behälter nach oben hin offen ausgebildet sein, um ein einfaches Einfüllen der reinigungsaktiven und/oder desinfizierenden Substanz zu ermöglichen und zudem dann, wenn ein automatisiertes Verdünnen (Aktivieren) der reinigungsaktiven und/oder desinfizierenden Substanz vorgesehen ist, eine einfache Automatisierung dieses Vorganges zu ermöglichen.

Gemäss einem weiteren Aspekt der Erfindung ist vorgesehen, dass das Verfahren zumindest nach Durchführung der vorbereitenden Verfahrensschritte automatisiert durchgeführt wird.

Um das oben diskutierte (aus dem Stand der Technik bekannte) Problem, dass nicht das gesamte milchführende System gereinigt und/oder desinfiziert wird, zu umgehen, ist in dem vorbereitenden Verfahrensschritt zumindest ein manuelles Trennen der Milchzufuhrleitung vom Milchbehälter und ein Aufstecken (Verbinden) der Milchzufuhrleitung an einen entsprechenden Anschluss des ersten Behälters vorgesehen. Indem jedoch danach das eigentliche Reinigungs-/ Desinfektionsverfahren und Spülverfahren gemäss diesem Aspekt der Erfindung automatisiert durchgeführt wird, ist die Benutzerinteraktion auf das notwendige Mass verringert, und der Reinigungs- bzw. Desinfektionsablauf wird vereinfacht. Gleichzeitig sind ein vollständiges Reinigen und/oder Desinfizieren sowie das zugehörige Spülen des vollständigen milchführenden Systems möglich. Gemäss einem weiteren Aspekt der Erfindung ist vorgesehen, dass ein Umschaltventil vorgesehen ist, welches mittels Ansteuerung mindestens die folgenden Stellungen annehmen kann:
- Verbindung einer Fluidfördereinrichtung, vorzugsweise einer mit einer Wasserzufuhr in Verbindung stehenden Pumpen-Heizeinrichtungs-Kombination, mit dem Fluideinlass; und
- Verbindung der Fluidfördereinrichtung mit dem Lufteinlass und Verschluss des Fluideinlasses.

Hierbei wird das Umschaltventil zum Durchführen des Verfahrensschrittes, wonach Fluid zu dem Fluideinlass geführt wird und der Lufteinlass belüftet wird, automatisch in die erstgenannte Stellung gebracht, wonach die Fluidfördereinrichtung mit dem Fluideinlass verbunden ist.

Um dann den Verfahrensschritt des Spülens durchzuführen, d. h. den Verfahrensschritt, wonach Wasser zu dem Lufteinlass geführt wird und der Fluideinlass verschlossen wird, wird dann das Umschaltventil automatisch in die letztgenannte Stellung gebracht, d. h. in die Stellung, in welcher die Fluidfördereinrichtung mit dem Lufteinlass verbunden ist und der Fluideinlass verschlossen ist.

Durch ein derartiges Umschaltventil, welches automatisch in verschiedene Stellungen gebracht werden kann, ist auf besonders einfache Weise eine automatisierte Durchführung des erfindungsgemässen Verfahrens möglich..

Bei der Verwendung eines derartigen Umschaltventils kann es gemäss einem weiteren Aspekt der Erfindung vorgesehen sein, dass das Umschaltventil zusätzlich die folgende Stellung aufweist:
- Verbindung der Fluidfördereinrichtung mit einem in den ersten Behälter mündenden Wasserauslass.

Hierbei ist es vorgesehen, dass das Umschaltventil zum Durchführen des Verfahrensschrittes, wonach Wasser in den ersten Behälter zugeführt wird, um die reinigungsaktive und/oder desinfizierende Substanz zu aktivieren, automatisch in die genannte Stellung gebracht wird, in welcher die Fluidfördereinrichtung mit einem in den ersten Behälter mündenden Wasserauslass verbunden ist.

Wenn der erste Behälter mit einer zu aktivierenden reinigungsaktiven und/oder desinfizierenden Substanz befüllt ist, beispielsweise mit einem Reinigungskonzentrat oder einer Reinigungstablette, so kann durch ein Umschaltventil mit einer derartigen zusätzlichen Stellung auch der Verfahrensschritt des Aktivierens der reinigungsaktiven und/oder desinfizierenden Substanz erreicht werden.

Gemäss einem weiteren Aspekt der vorliegenden Erfindung ist eine Kaffeemaschine zum Herstellen von Kaffeemischgetränken vorgesehen, die ein milchführendes System aufweist, das seinerseits folgendes aufweist:
- eine Milchzubereitungseinrichtung mit einem Milchauslass sowie einem Fluideinlass, einem Lufteinlass und einem Milcheinlass, die zum Fördern von Milch vom Milcheinlass zum Milchauslass unter Verwendung des Venturi-Effekts ausgebildet ist; und
- eine Milchzufuhrleitung, die an einem Ende mit dem Milcheinlass verbunden ist und die an einem anderen Ende wahlweise mit einem Milchbehälter verbindbar ist.

Die Kaffeemaschine ist hierbei dazu ausgebildet, das oben beschriebene Verfahren zum Reinigen und/oder Desinfizieren des milchführenden Systems automatisiert durchzuführen.

In einer Weiterbildung der Kaffeemaschine weist diese einen Wasserauslass zur Abgabe von Wasser auf, welcher derart angeordnet ist, dass der erste Behälter über den Wasserauslass mit Wasser füllbar ist. Bei einer derart weitergebildeten Kaffeemaschine ist eine besonders einfache Automatisierung des gesamten Reinigungsablaufes möglich, wobei hier je nach verwendetem Reinigungsmittel ein besonders einfaches Aktivieren einer reinigungsaktiven und/oder desinfizierenden Substanz durch Zuführen von Wasser möglich ist.

Gemäss einem weiteren Aspekt der Erfindung weist die Kaffeemaschine eine Behälteraufnahme für einen integralen Kombinationsbehälter aus erstem Behälter und zweitem Behälter auf. Die Behälteraufnahme ist dabei dazu ausgebildet, den Kombinationsbehälter genau dann sicher aufzunehmen, wenn der erste Behälter im Mündungsbereich des Wasserauslasses platziert ist (d.h. derart, dass über den Wasserauslass Wasser in den ersten Behälter füllbar ist) und wenn der zweite Behälter im Mündungsbereich des Milchauslasses platziert ist (d.h. derart, dass ein Reinigungs- bzw. Desinfektionsfluid aus der Milchzubereitungseinrichtung über den Milchauslass in den zweiten Behälter einleitbar ist).

Mit anderen Worten: Die Kaffeemaschine weist durch entsprechende Formgebung oder dergleichen eine an den integralen Kombinationsbehälter angepasste Behälteraufnahme auf, welche es verhindert, dass eine Bedienperson den integralen Kombinationsbehältern falsch unter dem Auslauf platziert. Insbesondere kann hierfür zusätzlich eine Positionierhilfe an dem Behälter vorgesehen sein, was eine besonders sichere Verwendung gewährleistet.

Weiterhin kann die Kaffeemaschine mit einer Sensoreinrichtung ausgestattet sein, welche dazu ausgebildet ist, zu erkennen, ob der integrale Kombinationsbehälter von der Behälteraufnahme aufgenommen ist und sich an einer Position befindet, welche gewährleistet, dass Wasser über den Wasserauslasses in den ersten Behälter füllbar ist und/oder ein Reinigungs- bzw. Desinfektionsfluid aus der Milchzubereitungseinrichtung über den Milchauslass in den zweiten Behälter einleitbar ist. Eine derartige Sensoreinrichtung ermöglicht eine automatische Erkennung des Kombinationsbehälters und eine automatische Überprüfung, ob der Kombinationsbehälter korrekt positioniert ist, um eine Reinigung bzw. Desinfizierung des milchführenden Systems einwandfrei (wie vorgesehen) durchführen zu können. Eine fehlerhafte Bedienung der Kaffeemaschine während einer Durchführung des beschriebenen Verfahrens zum Reinigen und/oder Desinfizierenden des milchführenden Systems (z.B. ein von einer Bedienperson veranlasstes, unvorhergesehenes Entfernen des Kombinationsbehälters während einer Durchführung des beschriebenen Verfahrens zum Reinigen und/oder Desinfizieren des milchführenden Systems) kann so mittels der Sensoreinrichtung automatisch erkannt werden. Die Kaffeemaschine kann beispielsweise derart ausgebildet sein, dass die Durchführung des beschriebenen Verfahrens zum Reinigen und/oder Desinfizierenden des milchführenden Systems sofort gestoppt oder verhindert wird, sobald die Sensoreinrichtung eine fehlerhafte Positionierung des Kombinationsbehälters erkennt.

Nachfolgend wird eine Ausführungsform einer Kaffeemaschine zum Durchführen des erfindungsgemässen Verfahrens anhand der Zeichnungen näher erläutert.

Es zeigen:
- Fig. 1:: eine schematische Darstellung von Bestandteilen einer Kaffeemaschine inklusive einer Milchzubereitungseinrichtung mit einem automatisch ansteuerbaren Umschaltventil in einer zum Mischen geeigneten Stellung;
- Fig. 2:: die schematische Darstellung aus Fig. 1 mit dem Umschaltventil in einer zum Reinigen und Desinfizieren geeigneten Stellung;
- Fig. 3:: die schematische Darstellung aus den Figuren 1 und 2 mit dem Umschaltventil in einer Geschlossenstellung; und
- Fig. 4:: die schematische Darstellung aus den Figuren 1 bis 3 mit dem Umschaltventil in einer zum Spülen geeigneten Stellung.

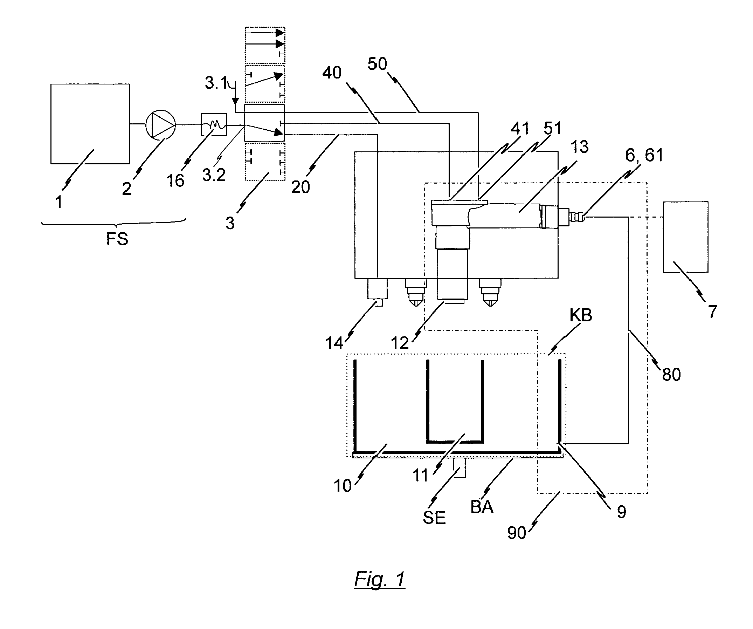

Fig. 1 zeigt die schematische Darstellung einiger Bestandteile einer nicht vollständig dargestellten Kaffeemaschine zum Bereiten von Kaffeemischgetränken gemäss einer Ausführungsform zum Durchführen des erfindungsgemässen Verfahrens.

Zentral ist eine Milchzubereitungseinrichtung 13 vorgesehen, welche in der Darstellung in Fig. 1 rechtsseitig einen Leitungsanschluss 6 für eine Milchzufuhrleitung 80 aufweist, wobei der Leitungsanschluss 6 gleichzeitig als Milcheinlass 61 in die Milchzubereitungseinrichtung 13 dient. Die Milchzubereitungseinrichtung 13 weist weiterhin einen Fluideinlass 41 sowie einen Lufteinlass 51 auf, die jeweils mit einer Fluidzufuhrleitung 40 bzw. einer Luftzufuhrleitung 50 verbunden sind. Ausgangsseitig weist die Milchzubereitungseinrichtung 13 einen Milchauslass 12 auf.

Die Milchzufuhrleitung 80 weist im vorliegenden Beispiel zwei Enden auf, wobei die Milchzufuhrleitung 80 an einem dieser Enden mit dem Leitungsanschluss 6 verbunden ist. Die Milchzufuhrleitung 80 ist am anderen dieser Enden mit einem Milchbehälter 7 verbunden, welcher gekühlt ausgebildet sein kann und die zuzubereitende Milch enthält.

Diese Verbindung mit dem Milchbehälter 7 ist trennbar ausgebildet, d. h. die Milchzufuhrleitung 80, welche gewöhnlicherweise als Schlauch ausgebildet ist, ist von einem entsprechenden Anschluss des Milchbehälters 7 abziehbar. Im aufgesteckten Zustand jedoch, welcher in Fig. 1 gestrichelt angedeutet ist, kann im Normalbetrieb der Kaffeemaschine beim Bezug von Milch, d. h. bei Zubereitung von Milch in der Milchzubereitungseinrichtung 13, die Milchzubereitungseinrichtung 13 an ihrem Fluideinlass 41 mit Dampf versorgt werden, sodass der Dampf die Milchzubereitungseinrichtung 13 durchströmt. Die Milchzubereitungseinrichtung 13 ist derart ausgebildet, dass in der Milchzubereitungseinrichtung 13 beim Durchströmen des Dampfes mittels des Venturi-Effekts ein Unterdruck erzeugt wird, welcher bewirkt, dass Milch aus dem Milchbehälter 7 durch die Milchzufuhrleitung 80 an den Milcheinlass 61 der Milchzubereitungseinrichtung 13 gesaugt wird, sich dort mit dem Dampf vermischt und dabei erhitzt wird. Sofern der Lufteinlass 51 belüftet ist, d.h. mit der Umgebungsatmosphäre in Verbindung steht, kann die Milch in der Milchzubereitungseinrichtung 13 ausser mit dem Dampf auch zusätzlich mit Luft gemischt und dadurch zu einem Milchschaum "aufgeschäumt" werden. Die auf diese Weise mittels des Dampfes erhitzte Milch bzw. der auf diese Weise erzeugte Milchschaum wird schliesslich am Milchauslass 12 aus der Milchzubereitungseinrichtung 13 ausgegeben.

Zum Durchführen des erfindungsgemässen Reinigungsverfahrens ist in der dargestellten Ausführungsform nun vorgesehen, dass die Fluidzufuhrleitung 40 sowie die Luftzufuhrleitung 50 mit einem Umschaltventil 3 verbunden sind, welches eingangsseitig einen ersten Eingang 3.1 aufweist, welcher mit der Umgebungsatmosphäre in Verbindung steht und somit eine (mithilfe des Umschaltventil 3 kontrollierbare) Zufuhr von Luft in die Luftzufuhrleitung 50 ermöglicht, sowie einen zweiten Eingang 3.2 aufweist, der mit einem fluidfördernden System FS (im Folgenden auch "Fluidfördereinrichtung FS" genannt) verbunden ist. Das fluidfördernde System FS (bzw. die "Fluidfördereinrichtung FS") ist in der dargestellten Ausführungsform gebildet durch einen Wassertank 1, einer damit verbundenen Pumpe 2 sowie einer nachgeordneten Heizeinrichtung 16. Mittels dieses fluidfördernden Systems FS kann wahlweise kaltes, warmes oder heisses Wasser oder auch Dampf dem zweiten Eingang 3.2 des Umschaltventils 3 zugeführt werden.

Das Umschaltventil 3 befindet sich in Fig. 1 in einer Position, in welcher es die Fluidzufuhrleitung 40 zur Milchzubereitungseinrichtung 13 verschliesst. Die Luftzufuhrleitung 50 der Milchzubereitungseinrichtung 13 steht über das Umschaltventil 3 mit der Umgebungsatmosphäre in Verbindung.

Ein weiterer Ausgang des Umschaltventils 3 ist über eine Wasserzufuhrleitung 20 mit einem Wasserauslass 14 der Kaffeemaschine verbunden, welcher in der dargestellten Ausführungsform in geringem Abstand neben dem Milchauslass 12 der Milchzubereitungseinrichtung 13 angeordnet ist.

Unterhalb des Milchauslasses 12 der Milchzubereitungseinrichtung 13 und unterhalb des Wasserauslasses 14 ist ein kombinierter Behälter KB (im Folgenden auch "Kombinationsbehälter KB" oder auch "Behälter KB" genannt) angeordnet, welcher einen ersten äusseren Behälter 10 sowie einen zweiten inneren Behälter 11 aufweist. Wie Fig. 1-4 andeuten, umfasst die Kaffeemaschine eine Behälteraufnahme BA für den kombinierten Behälter KB. Diese Behälteraufnahme BA ist derart ausgebildet, dass der kombinierte Behälter KB, wenn er von der Behälteraufnahme BA aufgenommen ist, jeweils in einer vorgegebenen Position relativ zur Milchzubereitungseinrichtung 13 angeordnet ist. Der erste Behälter 10 und der zweite Behälter 11 sind nach oben hin offen ausgebildet, stehen ansonsten jedoch nicht in einer Fluidverbindung miteinander. In der dargestellten Ausführungsform ist der kombinierte Behälter KB, bestehend aus erstem Behälter 10 und zweitem Behälter 11, mit einer (nicht dargestellten) Positionierungshilfe versehen, sodass eine Bedienperson den Behälter KB stets relativ zur Behälteraufnahme BA so anordnen wird wie in Fig. 1 dargestellt, nämlich mit dem Milchauslass 12 unmittelbar oberhalb des zweiten Behälters 11 und dem Wasserauslass 14 unmittelbar oberhalb des ersten Behälters 10.

Zum Initiieren des Reinigungs-/ Desinfektionsvorganges trennt eine Bedienperson die Milchzufuhrleitung 80 von dem Milchbehälter 7 und verbindet sie mit einem Leitungsanschluss 9, welcher am ersten Behälter 10 ausgebildet ist und mit diesem in Fluidverbindung steht. Im Folgenden wird davon ausgegangen, dass der erste Behälter 10 eine reinigungsaktive und/oder desinfizierende Substanz enthält (in Fig. 1-4 nicht dargestellt).

In der dargestellten Stellung des Umschaltventils 3 wird dann Wasser zum Aktivieren der reinigungsaktiven und/oder desinfizierende Substanz in den Behälter 10 automatisiert zugeführt, und zwar mittels der Pumpe 2 und der Heizeinrichtung 16 von dem Wassertank 1 durch das Umschaltventil 3 an den Wasserauslass 14. Während dieses Vorgangs ist die Heizeinrichtung 16 nicht eingeschaltet, d. h. der Behälter 10 wird bis zum Erreichen der gewünschten Konzentration mit kaltem oder lauwarmem Wasser befüllt. Sollte die Heizeinrichtung 16 vor diesem Vorgang auf eine Temperatur erhitzt worden sein, welche grösser als die Temperatur des dem Behälter 10 zugeführten Wassers ist, wird während dieses Vorgangs zusätzlich noch die Heizeinrichtung 16 gekühlt und ausserdem das dem Behälter 10 zugeführte Wasser erwärmt.

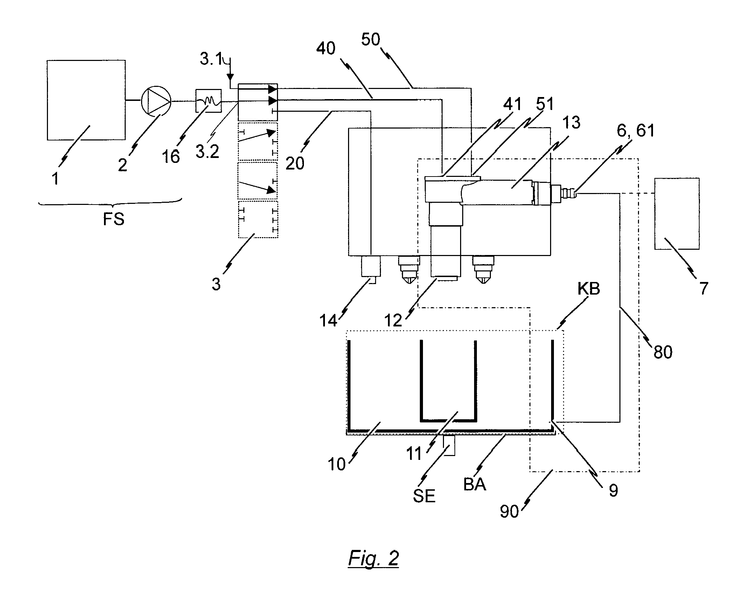

Sobald der erste Behälter 10 mit einer vorbestimmte Menge Wasser gefüllt ist, welche derart bemessen ist, dass aus der im ersten Behälter 10 befindlichen reinigungsaktiven und/oder desinfizierende Substanz und dem im ersten Behälter 10 enthaltenen Wasser ein zum Reinigen und/oder Desinfizieren des milchführenden Systems geeignetes Reinigungs- bzw. Desinfektionsfluid entsteht, wird das Umschaltventil 3 in die in Fig. 2 gezeigte Stellung automatisiert umgeschaltet. In dieser Stellung ist der Wasserauslass 14 über das Umschaltventil 3 gesperrt. Während die Pumpe 2 weiterhin Wasser aus dem Wassertank 1 fördert, wird nun die Heizeinrichtung 16 eingeschaltet, sodass das fluidfördernde System FS unter diesen Umständen dem Fluideinlass 41 Dampf über das Umschaltventil 3 und über die Fluidzufuhrleitung 40 zuführt (Fig. 2). Die Zufuhr von Dampf führt aufgrund des Venturi-Effekts zur Erzeugung eines Unterdrucks in der Milchzubereitungseinrichtung 13, sodass das Reinigungs- bzw. Desinfektionsfluid aus dem ersten Behälter 10 über die Milchzufuhrleitung 80 in den Milcheinlass 61 der Milchzubereitungseinrichtung 13 gesaugt wird. Dieses mit Milch kontaminierte Reinigungs- bzw. Desinfektionsfluid fliesst anschliessend über den Milchauslass 12 in den zweiten Behälter 11.

In der in Fig. 2 dargestellten Stellung des Umschaltventils 3 steht der Lufteinlass 51 der Milchzubereitungseinrichtung 13 weiterhin über das Umschaltventil 3 bzw. über den ersten Eingang 3.1 des Umschaltventils 3 mit der Umgebungsatmosphäre in Verbindung. Diese Verbindung mit der Umgebungsatmosphäre bewirkt im vorliegenden Beispiel während des vorstehend beschriebenen Ansaugens des Reinigungs- bzw. Desinfektionsfluids auch eine Zufuhr von Luft in die Milchzubereitungseinrichtung 13, sodass in der Milchzubereitungseinrichtung 13 das angesaugte Reinigungs- bzw. Desinfektionsfluid auch mit Luft gemischt wird. Es sei allerdings darauf hingewiesen, dass diese Zufuhr von Luft für die vorstehend beschriebene Reinigung bzw. Desinfektion der Milchzubereitungseinrichtung 13 nicht notwendig ist. Dementsprechend wäre es auch denkbar, anstelle des in Fig. 1-4 dargestellten Umschaltventils 3 ein anderes Ventil zu verwenden, welches zum Zwecke des Ansaugens des Reinigungs- bzw. Desinfektionsfluids das fluidfördernde System FS mit der Fluidzufuhrleitung 40 verbindet (wie in Fig. 2 dargestellt), allerdings (alternativ zu der in Fig. 2 dargestellten Situation) die Luftzufuhrleitung 50 gegenüber dem ersten Eingang 3.1 des Umschaltventils 3 dicht verschliesst, sodass keine Zufuhr von Luft vom ersten Eingang 3.1 zum Lufteinlass 51 erfolgen kann.

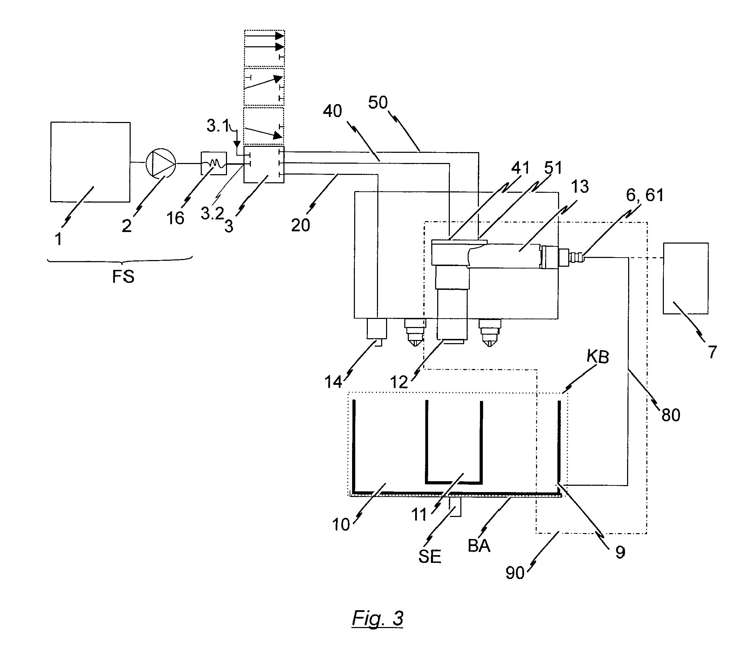

Sobald genügend Reinigungs- bzw. Desinfektionsfluid aus dem ersten Behälter 10 durch die Milchzubereitungseinrichtung 13 geflossen ist, wird die Pumpe 2 gestoppt und das Umschaltventil 3 in eine allseitig geschlossene Stellung gebracht, welche in Fig. 3 gezeigt ist.

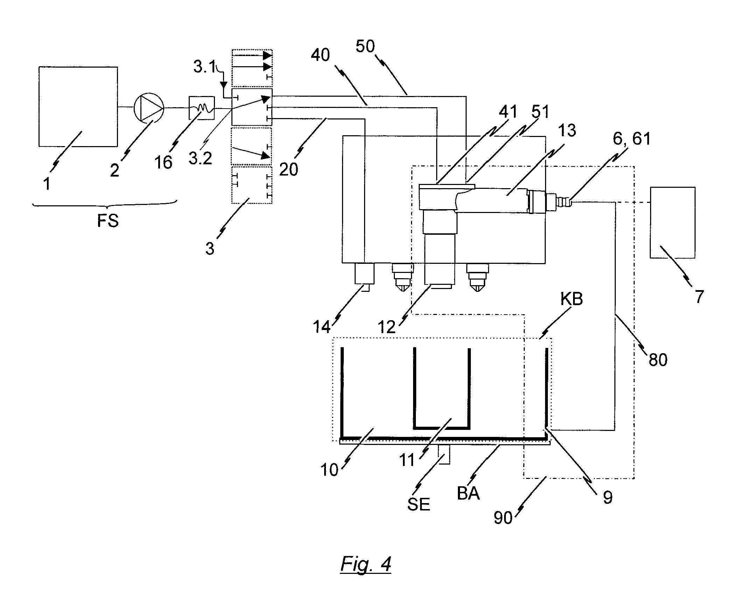

Anschliessend wird das Umschaltventil in die in Fig. 4 gezeigte Stellung gebracht, um im Anschluss an das vorstehend beschriebene Reinigen und/oder Desinfizieren des milchführenden Systems 90 zu ermöglichen, dass im milchführenden System 90 möglicherweise vorhandene Rückstände des Reinigungs- bzw. Desinfektionsfluids beseitigt werden können. Hierbei ist nunmehr nicht der Fluideinlass 41 der Milchzubereitungseinrichtung 13 mit dem fluidfördernden System FS verbunden; vielmehr wird über die Luftzufuhrleitung 50 der Lufteinlass 51 der Milchzubereitungseinrichtung 13 über das Umschaltventil 3 mit Wassertank 1, Pumpe 2 und Heizeinrichtung 16 verbunden. Anschliessend wird klares Wasser (Spülwasser) aus dem Wassertank 1 (vorzugsweise Trinkwasser) zum Lufteinlass 51 der Milchzubereitungseinrichtung 13 gefördert, während der Fluideinlass 41 über das Umschaltventil 3 verschlossen ist.

Das über den Lufteinlass 51 in die Milchzubereitungseinrichtung 13 eintretende Spülwasser teilt sich bezogen auf seinen Fliessweg auf und fliesst sowohl über den Milchauslass 12 in den zweiten Behälter 11 als auch über die Milchzufuhrleitung 80 in den ersten Behälter 10. Hierbei spült das Spülwasser sowohl das Innere der Milchzubereitungseinrichtung 13 inklusive Milchauslass 12 als auch vollständig die Milchzufuhrleitung 80. Zum Entleeren des gesamten Kreislaufes kann zum Abschluss des Spülvorganges durch Aktivierung der Heizeinrichtung 16 ein Dampfstoss durch den Milchauslass 12 sowie die Milchzufuhrleitung 80 geleitet werden, sodass die in diesem System verbliebene Spülflüssigkeit weitestgehend ausgetrieben wird.

Alternativ zu der vorstehend genannten Vorgehensweise wäre es auch möglich, zum Reinigen und/oder Desinfizieren des milchführenden Systems 90 in dem ersten Behälter 10 eine reinigungsaktive und/oder desinfizierende Substanz in Form eines Reinigungs- bzw. Desinfektionsfluids bereitzustellen, welches sich bereits in einem gebrauchsfertigen Zustand befindet und nicht noch zusätzlich mit Wasser vermischt werden muss. In diesem Fall kann (abweichend von der im Zusammenhang mit Fig. 1 dargestellten Vorgehensweise) darauf verzichtet werden, zu Beginn des Verfahrens Wasser aus dem Wasserauslass 14 in den ersten Behälter 10 einzuleiten. Stattdessen steht das in dem ersten Behälter 10 bereitgestellte Reinigungs- bzw. Desinfektionsfluids in seiner bereits vorliegenden Form zu Reinigen und/oder Desinfizieren des milchführenden Systems 90 zur Verfügung und kann unverändert - wie im Zusammenhang mit Fig. 2 beschrieben - aus dem ersten Behälter 10 über die Milchzufuhrleitung 80 in die Milchzubereitungseinrichtung 13 eingeleitet werden.

Während des Reinigungsvorganges ist das frische Reinigungs- bzw. Desinfektionsfluid stets von dem kontaminierten Reinigungs- bzw. Desinfektionsfluid getrennt. Hierdurch ergibt sich eine sichere Reinigung, welche durch die Verbindung der Milchzufuhrleitung 80 mit dem Leitungsanschluss 9 des ersten Behälters 10 bezogen auf das gesamte milchführende System 90 durchgeführt werden kann.

Wie Fig. 1-4 weiterhin andeuten, kann die Kaffeemaschine mit einer Sensoreinrichtung SE ausgestattet sein, welche dazu ausgebildet ist, zu erkennen, ob der kombinierte Behälter KB von der Behälteraufnahme BA aufgenommen ist und sich derart an einer vorgegebenen Position relativ zur Milchzubereitungseinrichtung 13 befindet, dass Wasser über den Wasserauslasses 14 in den ersten Behälter 10 füllbar ist und/oder ein Reinigungs- bzw. Desinfektionsfluid aus der Milchzubereitungseinrichtung 13 über den Milchauslass 12 in den zweiten Behälter 11 einleitbar ist. Die Sensoreinrichtung SE kann zu diesem Zweck mit konventionellen, für diesen Zweck geeigneten Sensoren (z.B. optischen, mechanischen, magnetischen, piezoelektrischen Sensoren oder dergleichen) ausgestattet sein. Dementsprechend kann die Sensoreinrichtung SE dazu ausgebildet sein, Signale zu erzeugen, welche angeben, ob sich der kombinierte Behälter KB an der vorgegebenen Position relativ zur Milchzubereitungseinrichtung 13 (wie beschrieben) befindet. Die von der Sensoreinrichtung SE erzeugten Signale können schliesslich von einer (in Fig. 1-4 nicht dargestellten) Steuervorrichtung der Kaffeemaschine ausgewertet werden, um das vorstehend beschriebene Verfahren zum Reinigen und/oder Desinfizieren des milchführenden Systems 90 der Kaffeemaschine zu steuern. Es ist in diesem Zusammenhang vorausgesetzt, dass die Steuereinrichtung der Kaffeemaschine ausgebildet ist, die Pumpe 2, die Heizeinrichtung 16 und das Umschaltventil 3 entsprechend anzusteuern, um eine Durchführung der oben beschriebenen Verfahrensschritte hinsichtlich einer Zufuhr von Wasser, Dampf oder Luft in die Wasserzufuhrleitung 20, die der Fluidzufuhrleitung 40 oder die Luftzufuhrleitung 50 zu steuern. Eine fehlerhafte Platzierung des kombinierten Behälters KB kann von der Steuervorrichtung der Kaffeemaschine mithilfe der Sensoreinrichtung SE automatisch erkannt werden und somit bei der Steuerung der Kaffeemaschine während der Durchführung des Verfahren zum Reinigen und/oder Desinfizieren des milchführenden Systems 90 berücksichtigt werden. Die Steuervorrichtung kann beispielsweise eine Durchführung des Verfahrens zum Reinigen und/oder Desinfizieren des milchführenden Systems 90 in Abhängigkeit von Signalen der Sensoreinrichtung SE verhindern oder stoppen.

In den in Fig. 1-4 dargestellten Beispielen ist ein einziges Umschaltventil 3 vorgesehen, welches in mehrere Stellungen gebracht werden kann, um einerseits eine Fluidverbindung zwischen dem fluidfördernden System (bzw. der Fluidfördereinrichtung) FS und wahlweise entweder der Wasserzufuhrleitung 20 oder der Fluidzufuhrleitung 40 oder der Luftzufuhrleitung 50 (über den zweiten Eingang 3.2 des Umschaltventils 3) herstellen zu können und anderseits auch eine Verbindung zwischen der Luftzufuhrleitung 50 und der Umgebungsatmosphäre (über den ersten Eingang 3.1 des Umschaltventils 3) herstellen zu können. Es sei darauf hingewiesen, dass anstelle des einen Umschaltventils 3 auch mehrere separate, jeweils unabhängig voneinander steuerbare Ventile verwendet werden könnten, um die vorstehend genannten Fluidverbindungen der Wasserzufuhrleitung 20, der Fluidzufuhrleitung 40 und der Luftzufuhrleitung 50 mit dem fluidfördernden System FS und zusätzlich die Verbindung der Luftzufuhrleitung 50 mit der Umgebungsatmosphäre durch Öffnen oder Schliessen der jeweiligen Ventile zu steuern.

Es sei an dieser Stelle angemerkt, dass die Erfindung nicht auf die dargestellte Ausführungsform beschränkt ist. Abänderungen hiervon sind dem Fachmann geläufig.

## Patentansprüche

1. Verfahren zum Reinigen und/oder Desinfizieren eines milchführenden Systems (90) einer Kaffeemaschine zum Herstellen von Kaffeemischgetränken, wobei das milchführende System (90) Folgendes aufweist:
• eine Milchzubereitungseinrichtung (13) mit einem Milchauslass (12) sowie einem Fluideinlass (41), einem Lufteinlass (51) und einem Milcheinlass (61), die zum Fördern von Milch vom Milcheinlass (61) zum Milchauslass (12) unter Verwendung des Venturi-Effekts ausgebildet ist; und
• eine Milchzufuhrleitung (80), die an einem Ende mit dem Milcheinlass (61) verbunden ist und die an einem anderen Ende wahlweise mit einem Milchbehälter (7) verbindbar ist,
wobei das Verfahren die folgenden vorbereitenden Verfahrensschritte aufweist:
• Trennen der Milchzufuhrleitung (80) vom Milchbehälter (7) und Verbinden der Milchzufuhrleitung (80) mit einem ersten Behälter (10), der eine reinigungsaktive und/oder desinfizierende Substanz enthält; und
• Positionieren eines zweiten Behälters (11) unterhalb des Milchauslasses (12),
und wobei das Verfahren die folgenden weiteren Verfahrensschritte in dieser Reihenfolge aufweist:
i) Zuführen von Fluid, vorzugsweise von Dampf, zu dem Fluideinlass (41), um die reinigungsaktive und/oder desinfizierende Substanz durch die Milchzufuhrleitung (80) und den Milchauslass (12) in den zweiten Behälter (11) zu fördern, um so das milchführende System (90) zu reinigen und/oder zu desinfizieren; und
j) Zuführen von Wasser zu dem Lufteinlass (51) und Verschliessen des Fluideinlasses (41), um Wasser durch die Milchzufuhrleitung (80) in den ersten Behälter (10) und um Wasser durch den Milchauslass (12) in den zweiten Behälter (11) zu fördern, um so das milchführende System (90) zu spülen.

2. Verfahren nach Anspruch 1, wobei das Verfahren nach dem Verfahrensschritt j) den folgenden Verfahrensschritt aufweist:
k) Zuführen, insbesondere stossweises Zuführen, von Dampf zu dem Lufteinlass (51) und Verschliessen des Fluideinlasses (41), um das Wasser aus der Milchzufuhrleitung (80) und dem Milchauslass (12) auszutreiben.

3. Verfahren nach Anspruch 1 oder 2 wobei das Verfahren vor dem Verfahrensschritt i) den folgenden Verfahrensschritt aufweist:
h) Zuführen von Wasser in den ersten Behälter (10), um die reinigungsaktive und/oder desinfizierende Substanz zu aktivieren.

4. Verfahren nach einem der Ansprüche 1 bis 3, wobei der erste Behälter (10) und der zweite Behälter (11) ineinander geschachtelt und vorzugweise integral ausgebildet sind.

5. Verfahren nach einem der Ansprüche 1 bis 4, wobei das Verfahren zumindest nach Durchführung der vorbereitenden Verfahrensschritte automatisiert durchgeführt wird.

6. Verfahren nach einem der Ansprüche 1 bis 5, wobei ein Umschaltventil (3) vorgesehen ist, das mittels Ansteuerung mindestens die folgenden Stellungen annehmen kann:
a) Verbindung einer Fluidfördereinrichtung (FS), vorzugsweise einer mit einer Wasserzufuhr in Verbindung stehenden Pumpen-Heizeinrichtungs-Kombination (2, 16), mit dem Fluideinlass (41); und
b) Verbindung der Fluidfördereinrichtung (FS) mit dem Lufteinlass (51) und Verschluss des Fluideinlasses (41),
wobei das Umschaltventil (3) zum Durchführen des Verfahrensschritts i) automatisch in die Stellung a) gebracht wird, und wobei das Umschaltventil (3) zum Durchführen des Verfahrensschritts j) automatisch in die Stellung b) gebracht wird.

7. Verfahren nach Anspruch 6, wobei das Umschaltventil (3) zusätzlich die folgende Stellung aufweist:
c) Verbindung der Fluidfördereinrichtung (FS) mit einem in den ersten Behälter (10) mündenden Wasserauslass (14),
wobei das Umschaltventil (3) zum Durchführen des Verfahrensschrittes h) automatisch in die Stellung c) gebracht wird.

8. Kaffeemaschine zum Herstellen von Kaffeemischgetränken, die ein milchführendes System (90) aufweist, das seinerseits Folgendes aufweist:
• eine Milchzubereitungseinrichtung (13) mit einem Milchauslass (12) sowie einem Fluideinlass (41), einem Lufteinlass (51) und einem Milcheinlass (61), die zum Fördern von Milch vom Milcheinlass (61) zum Milchauslass (12) unter Verwendung des Venturi-Effekts ausgebildet ist; und
• eine Milchzufuhrleitung (80), die an einem Ende mit dem Milcheinlass (61) verbunden ist und die an einem anderen Ende wahlweise mit einem Milchbehälter (7) verbindbar ist,
wobei die Kaffeemaschine dazu ausgebildet ist, das Verfahren zum Reinigen und/oder Desinfizieren des milchführenden Systems (90) nach einem der Ansprüche 1 bis 7 automatisiert durchzuführen.

9. Kaffeemaschine nach Anspruch 8, wobei die Kaffeemaschine ferner einen Wasserauslass (14) zur Abgabe von Wasser aufweist, welcher derart angeordnet ist, dass der erste Behälter (10) über den Wasserauslass (14) mit Wasser füllbar ist.

10. Kaffeemaschine nach Anspruch 8, wobei die Kaffeemaschine eine Behälteraufnahme (BA) für einen integralen Kombinationsbehälter (KB) aus erstem Behälter (10) und zweitem Behälter (11) aufweist, wobei die Behälteraufnahme dazu ausgebildet ist, den Kombinationsbehälter (KB) genau dann sicher aufzunehmen, wenn der erste Behälter (10) im Mündungsbereich des Wasserauslasses (14) platziert ist und wenn der zweite Behälter (11) im Mündungsbereich des Milchauslasses (12) platziert ist.

11. Kaffeemaschine nach Anspruch 10,
mit einer Sensoreinrichtung (SE), welche dazu ausgebildet ist, zu erkennen, ob der integrale Kombinationsbehälter (KB) von der Behälteraufnahme (BA) aufgenommen ist und sich an einer Position befindet, welche gewährleistet, dass Wasser über den Wasserauslass (14) in den ersten Behälter (10) füllbar ist und/oder ein Reinigungs- bzw. Desinfektionsfluid aus der Milchzubereitungseinrichtung (13) über den Milchauslass (12) in den zweiten Behälter (11) einleitbar ist.
